# EUROPEAN PATENT SPECIFICATION

(11) **EP 0 670 370 B1**
(45) Date of publication and mention of the grant of the patent: **09.04.2003**
(21) Application number: 95100259.1
(22) Date of filing: 10.01.1995
(51) Int. Cl.: C12N 15/52, C12P 13/14, C12N 1/21

(54) **Method of producing L-glutamic acid by fermentation**
Verfahren zur Herstellung von Glutaminsäure durch Fermentation
Procédé de préparation de l'acide glutamique par fermentation

(30) Priority: 10.01.1994 JP 82594
(43) Date of publication of application: 06.09.1995
(73) Proprietor: Ajinomoto Co., Inc., Tokyo 104 (JP)
(72) Inventor: Ono, Eiji, c/o Central Research Laboratories, Kawasaki-ku, Kawasaki-shi, Kanagawa-ken (JP); Tsujimoto, Nobuharu, c/o Central Research Lab., Kawasaki-ku, Kawasaki-shi, Kanagawa-ken (JP); Matsui, Kazuhiko, c/o Central Research Lab., Kawasaki-ku, Kawasaki-shi, Kanagawa-ken (JP); Kurahashi, Osamu, c/o Central Research Lab., Kawasaki-ku, Kawasaki-shi, Kanagawa-ken (JP)
(74) Representative: Strehl Schübel-Hopf & Partner

(56) References cited:
- EP-A- 0 143 195
- FR-A- 2 575 492
- FR-A- 2 680 178

## Description

### Field of the Invention

The present invention relates to a mutant useful for producing L-glutamic acid by fermentation as well as a method of producing L-glutamic acid by fermentation using such a mutant. L-glutamic acid is an amino acid widely used as an additive for foods and in medicaments.

### Prior Art

L-glutamic acid has conventionally been produced by fermentation using glutamic acid-producing bacteria and mutants thereof such as those of the genus Brevibacterium, Corynebacterium or Microbacterium (Amino acid fermentation, Gakkai Shuppan Center, pp.195 to 215 (1986)). Other known methods of producing L-glutamic acid by fermentation include a method employing microorganisms of the genus Bacillus, Streptomyces or Penicillium (US Patent No. 3,220,929) and a method employing microorganisms of the genus Pseudomonas, Arthrobacter, Serratia or Candida (US Patent No. 3,563,857). Even though such conventional methods produce significantly large amounts of L-glutamic acid, an even more efficient and less expensive method of producing L-glutamic acid is desired in order to meet the ever-increasing demand.

Escherichia coli is a potentially excellent L-glutamic acid-producing bacterium in view of its high growth rate and the availability of sufficient gene information, while the reported amount of L-glutamic acid production by Escherichia coli is as low as 2.3 g/l (J. Biochem., Vol. 50, pp.164 to 165 (1961)). Recently, a mutant exhibiting a deficient or reduced α-ketoglutarate dehydrogenase (hereinafter referred to as α-KGDH) was reported to have the ability to produce large amounts of L-glutamic acid (French Patent Application Laid-Open No. 2680178).

### Problems to be Solved by the Invention

An objective of the present invention is to enhance the L-glutamic acid-producing ability of strains belonging to the genus Escherichia and to provide a method of producing L-glutamic acid more efficiently and at a lower cost.

### Means to Solve the Problems

Now it has been found surprisingly in our study on the production of L-glutamic acid by mutants of Escherichia coli that a mutant whose α-KGDH activity is deficient or reduced, and whose phosphoenolpyruvate carboxylase (hereinafter referred to as PPC) and glutamate dehydrogenase (hereinafter referred to as GDH) activities are enhanced, has a high L-glutamic acid-producing ability, and thus the present invention has been accomplished.

Accordingly, the present invention relates to :

A mutant of the genus Escherichia having L-glutamic acid-producing ability whose α-KGDH activity is deficient or reduced, and PPC and GDH activities are enhanced; and,

A method of producing L-glutamic acid by fermentation comprising culturing in a liquid culture medium a mutant of the genus Escherichia having L-glutamic acid-producing ability whose α-KGDH activity is deficient or reduced and PPC and GDH activities are enhanced, accumulating L-glutamic acid in the culture, and recovering L-glutamic acid therefrom.

The present invention is described in more detail below.

### (1) Derivation of a mutant of the genus Escherichia exhibiting deficient or reduced α-KGDH activity

As a starting parent strain to be used for preparing the present mutant, any non-pathogenic strain of the genus Escherichia may be employed. Examples of such strains are listed below.
Escherichia coli K-12 (ATCC 10798)
Escherichia coli W3110 (ATCC 27325)
Escherichia coli B (ATCC 11303)
Escherichia coli W (ATCC 9637)

A mutant of the genus Escherichia which has L-glutamic acid-producing ability and having deficient or reduced α-KGDH activity may be prepared as follows.

The starting parent strain mentioned above is first exposed to X-radiation or ultraviolet light or mutagenic agents such as N-methyl-N'-nitro-N-nitrosoguanidine (hereinafter referred to as NG) to introduce the mutation.

Alternatively, gene engineering technology, for example, gene recombination, gene transformation or cell fusion, may be used to efficiently introduce the intended mutation.

A method of obtaining an α-KGDH-deficient mutant by means of gene recombination is conducted as follows. Based on the known nucleotide sequence (Euro. J. Biochem. Vol. 141, pp. 351 to 359 (1984)) of α-ketoglutarate dehydrogenase gene (hereinafter referred to as sucA gene), primers are synthesized and then the sucA gene is amplified by the PCR method using the chromosomal DNA as a template. Into the amplified the sucA gene, a drug-resistant gene is inserted to obtain a sucA gene whose function is lost. Subsequently, using homologous recombination, the sucA gene on the chromosome is replaced by a sucA gene whose function is lost by means of the insertion of the drug-resistant gene.

After subjecting the parent strain to mutagenic treatment, the intended mutants may be screened by procedures as illustrated below.

A mutant exhibiting a deficient or reduced α-KGDH activity is either not able to grow or is able to grow only at a significantly reduced growth rate in a minimum culture medium containing glucose as the carbon source under aerobic condition. However, even under such condition, normal growth is possible by adding succinic acid or lysine plus methionine to the minimum culture medium containing glucose. On the other hand, anaerobic condition allows the mutant to grow even in the minimum culture medium containing glucose (Molec. Gen. Genetics, Vol. 105, pp. 182 to 190 (1969)). Based on these findings, the desired mutants can be screened.

The following strain is an example of the mutants thus obtained whose α-KGDH activity is deficient or reduced and which are listed below.
Escherichia coli W3110 sucA::Km^{r}

A mutant whose α-KGDH activity is deficient or reduced is more useful in view of its enhanced ability to produce L-glutamic acid when it further has the properties that L-glutamic acid-degrading activity is reduced or the expression of ace operon, that is, malate synthase (aceB) - isocitrate lyase (aceA) - isocitrate dehydrogenase kinase/phosphatase (aceK) operon becomes constitutive. These properties are discussed in French Patent Application Laid-open No. 2680178. As a matter of course, properties already known to be effective for improving L-glutamic acid-productivity, such as various types of auxotrophy, antimetabolite resistance and antimetabolite sensitivity, are also desirable for enhancing L-glutamic acid production ability.

A mutant having reduced ability to degrade L-glutamic acid may be isolated as a mutant which either cannot grow or can grow only slightly in a minimum culture medium containing L-glutamic acid as the sole carbon source instead of glucose or containing L-glutamic acid as a sole nitrogen source instead of ammonium sulfate. However, as a matter of course, when an auxotroph is employed for the derivation, the minimum essential amount of the nutrient required for the growth may be added to the culture medium.

A mutant in which the expression of the ace operon is constitutive may be obtained as a strain whose parent strain is a phosphoenolpyruvate synthase-deficient strain and which can grow in a minimum culture medium containing lactic acid as the carbon source but cannot grow in a minimum culture medium containing pyruvic acid or acetic/pyruvic acid as the carbon source, or as a strain which shows a higher growth rate than that of its parent strain whose α-KGDH is deficient or reduced under aerobic condition (J. Bacteriol., Vol. 96, pp. 2185 to 2186 (1968)).

Examples of the mutants described above are as follows.
Escherichia coli AJ 12628 (FERM BP-3854)
Escherichia coli AJ 12624 (FERM BP-3853)
Escherichia coli AJ 12628 is a mutant having a reduced α-KGDH activity and a reduced ability to degrade L-glutamic acid in combination with constitutive expression of ace operon. Escherichia coli AJ 12624 is a mutant having reduced α-KGDH activity and a reduced ability to degrade L-glutamic acid (French Patent Application Laid-open No. 2680178).

In the mutant thus obtained which exhibits deficient or reduced α-KGDH activity, the flow of biosynthesis of L-glutamic acid via a -ketoglutaric acid in the TCA cycle is improved, resulting in an enhanced ability of producing L-glutamic acid. Also the productivity of L-glutamic acid is increased in the mutant exhibiting deficient or reduced α-KGDH activity and significantly low ability to degrade the produced L-glutamic acid or in the mutant further having a constitutive expression of the ace operon whereby the growth is improved.

### (2) Derivation of a mutant of the genus Escherichia having amplified PPC activity and GDH activity

In the examples described below, a mutant of the genus Escherichia having amplified PPC and GDH activities was obtained from a starting parent strain exhibiting deficient or reduced α-KGDH activity and having the ability to produce L-glutamic acid. It is also possible to use a wild strain of the genus Escherichia as the parent strain to obtain a mutant having amplified PPC and GDH activities whereafter a mutant is bred which exhibits deficient or reduced α-KGDH activity.

Accordingly, the starting parent strain used to prepare a mutant having amplified PPC and GDH activities is preferably a mutant of the genus Escherichia whose α-KGDH activity is deficient or reduced and which has the ability to produce L-glutamic acid or a non-pathogenic wild type strain of the genus Escherichia. Examples of such strains are listed below.
Escherichia coli W3100 sucA::Km^{r}
Escherichia coli AJ 12628 (PERM BP-3854)
Escherichia coli AJ 12624 (FERM BP-3853) (Those listed above are the mutants of the genus Escherichia whose α -KGDH activity is deficient or reduced and which have the ability to produce L-glutamic acid.)
Escherichia coli K-12 (ATCC 10798)
Escherichia coli W3110 (ATCC 27325)
Escherichia coli B (ATCC 11303)
Escherichia coli W (ATCC 9637)
(Those listed above are the non-pathogenic wild strains of the genus Escherichia.)

In order to amplify PPC and GDH activities, the genes coding for PPC and GDH are cloned in an appropriated plasmid, which is then used to transform the starting parent strain employed as a host. The copies of the genes coding for PPC and GDH (hereinafter referred to as ppc gene and gdhA gene, respectively) in the transformed cells are increased, resulting in amplified PPC and GDH activities.

The ppc gene and gdhA gene to be cloned may be cloned into a single plasmid to be introduced into the starting parent strain employed as the host, or may be cloned separately into two types of plasmid which are compatible in the starting parent strain.

Alternatively, the amplification of PPC and GDH activities may be conducted by allowing the ppc and gdhA genes to be present as multicopies on the chromosomal DNA of the starting parent strain employed as the host. In order to introduce the ppc and gdhA genes as multicopies into the chromosomal DNA of the genus Escherichia, homologous recombination is applied utilizing a target sequence present as a multicopy on the chromosomal DNA. The sequence present as the multicopy may be a repetitive DNA and an inverted repeat present at the terminal of insertion sequence. Alternatively, as described in Japanese Patent Application Laid-open No. 2-109985, the ppc and gdhA genes are cloned on a transposon, which is then transposed, thereby introducing the multicopy into the chromosomal DNA. The copies of the ppc and gdhA genes in the transformed cells are increased, resulting in the amplification of PPC and GDH activities.

In addition to the gene amplification described above, the amplification of PPC and GDH activities may also be conducted by replacing the promoters of the ppc and gdhA genes with those having higher potencies. For example, lac promoter, trp promoter, trc promoter, tac promoter, P_{R} promoter and P_{L} promoter of a lambda phage are known to be strong promoters. By enhancing the expression of the ppc gene and of the gdhA gene, the PPC and GDH activities are amplified.

The ppc and gdhA genes can be obtained by isolating the genes which are complementary with regard to auxotrophy of the mutants which are either PPC or GDH deficient. Alternatively, since the nucleotide sequences of these genes of Escherichia coli are known (J. Biochem., Vol. 95, pp. 909 to 916 (1984); Gene, Vol. 27, pp. 193 to 199 (1984)), the primers are synthesized based on the nucleotide sequences and then the genes are obtained by the PCR method using the chromosomal DNA as the template.

### (3) Production of L-glutamic acid by fermentation using a mutant of the genus Escherichia capable of producing L-glutamic acid which exhibits deficient or reduced α-KGDH activity and has amplified PPC and GDH activities

For the purpose of producing L-glutamic acid by fermentation using a mutant of the genus Escherichia capable of producing L-glutamic acid which exhibits deficient or reduced α-KGDH activity and has amplified PPC and GDH activities, a standard culture medium containing carbon sources, nitrogen sources, inorganic salts and, if necessary, organic trace nutrients such as amino acids and vitamins and a standard culture method may be employed. The carbon sources and the nitrogen sources employed in the culture medium may be any of those catabolized by the mutant employed.

The carbon sources may be saccharides such as glucose, glycerol, fructose, sucrose, maltose, mannose, galactose, starch hydrolysate and molasses, and organic acids such as acetic acid and citric acid may also be employed independently or in combination with other carbon sources.

The nitrogen sources may be ammonia and ammonium salts such as ammonium sulfate, ammonium carbonate, ammonium chloride, ammonium phosphate, ammonium acetate as well as nitrates.

The organic trace nutrients may be amino acids, vitamins, fatty acids and nucleic acids as they are or as contained in peptone, casamino acid, yeast extract, soy protein hydrolysate and the like. In cases of using an auxotroph the nutrient required for its growth should be supplemented.

The inorganic salts may be phosphate, magnesium salts, calcium salts, iron salts, manganese salts and the like.

Cultivation is conducted at a fermentation temperature from 20 to 45°C at a pH controlled to be in a range of from 5 to 9 with aeration. When the pH is controlled during the cultivation, calcium carbonate or alkali such as ammonia gas may be added for neutralization. After culturing for from 10 hours to 4 days, a significant amount of L-glutamic acid is accumulated in the culture medium.

L-glutamic acid in the culture medium after cultivation may be recovered by any of the known methods. For example, the cells are removed from the culture medium, which is then concentrated and precipitated or subjected to ion exchange chromatography to obtain L-glutamic acid.

### Brief Description of the Drawings

- Fig. 1: shows the construction procedure of pBR-sucAB,
- Fig. 2: shows a procedure for disrupting the sucA gene on the chromosomal DNA of Escherichia coli W3110, and
- Fig. 3: shows the construction procedure of pGK.

### Examples

The present invention is further described by the following examples.

### Example 1

### (1) Cloning of sucA gene and dihydrolipoamide succinyl transferase gene of Escherichia coli

The nucleotide sequences of sucA gene and dihydrolipoamide succinyl transferase gene (hereinafter referred to as sucB gene) of Escherichia coli K12 are known. The known nucleotide sequences of sucA gene and sucB gene are disclosed in Euro. J. Biochem., Vol. 141, pp. 351 to 374 (1984), and also shown here as Sequ ID No. 7 in the sequence listing. The nucleotide sequence from the 327th through the 3128th base residues corresponds to ORF (open reading frame) of the sucA gene, while that from the 3143rd through the 4357th base residues corresponds to ORF of the sucB gene. According to the nucleotide sequences reported, primers shown in Sequ ID No.1 to 4 were synthesized and sucA and sucB genes were amplified by PCR method employing the chromosomal DNA of Escherichia coli W3110 as a template.

The synthetic primers used to amplify the sucA gene had the nucleotide sequences shown in Sequ ID No.1 and 2, and Sequ ID No.1 corresponds to the sequence consisting of the 45th through the 65th base residues in the nucleotide sequence figure of the sucA gene described in Euro. J. Biochem., Vol. 141, p. 354 (1984). It also corresponds to the sequence consisting of the 45th through the 65th base residues of the nucleotide sequence shown as Sequ ID No. 7.

Sequ ID No. 2 corresponds to the sequence consisting of the 3173rd through the 3193rd base residues in the nucleotide sequence figure of the sucB gene shown in Euro. J. Biochem., Vol. 141, p. 364 (1984). It also corresponds to the sequence consisting of the 3173rd through the 3193rd base residues of the nucleotide sequence shown as Sequ ID No. 7.

The synthetic primers used to amplify the sucB gene had the nucleotide sequences shown in Sequ ID No.3 and 4, and Sequ ID No. 3 corresponds to the sequence consisting of the 2179th through 2198th base residues in the nucleotide sequence figure of the sucA gene shown in Euro. J. Biochem., Vol. 141, p. 354 (1984). It also corresponds to the sequence consisting of the 2179th through the 2198th base residues of the nucleotide sequence shown as Sequ No. 7.

Sequ ID No. 4 corresponds to the sequence consisting of the 4566th through the 4591st base residues in the nucleotide sequence figure of the sucB gene shown in Euro. J. Biochem., Vol. 141, p. 364 (1984). It also corresponds to the sequence consisting of the 4566th through the 4591st base residues of the nucleotide sequence shown as Sequ ID No. 7. The sucA gene and the sucB gene form an operon.

The chromosomal DNA of Escherichia coli W3110 was recovered by a standard method (Seibutsukogaku Jikkensho, Ed. by Nippon Seibutsu Kogaku Kai, pp. 97 to 98, Baifukan (1992)).

The PCR reaction was carried out under the standard conditions described on page 8 of PCR Technology (Ed. by Henry Erlich, Stockton Press (1989)).

Both ends of PCR products thus produced were converted into blunt ends using T4 DNA polymerase and cloned into a vector pBR322 at the EcoRV site. The plasmid obtained by cloning the sucA gene into pBR322 was designated as pBR-sucA, and that constructed with sucB was designated as pBR-sucB. The plasmids thus obtained were introduced into Escherichia coli JM109 and the plasmids were prepared. Then the restriction maps were constructed and compared with the restriction maps of the sucA and sucB genes reported, thereby confirming that the genes which had been cloned were the sucA and sucB genes.

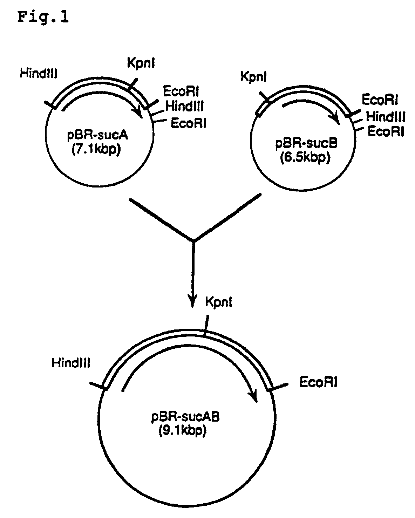

As shown in Fig. 1, pBR-sucB was digested with KpnI and EcoRI to prepare a DNA fragment containing the sucB gene. pBR-sucA was digested with KpnI and EcoRI to prepare a large fragment. Both fragments were ligated using T4 DNA ligase to produce pBR-sucAB.

### (2) Disruption of the sucA gene on chromosomal DNA of Escherichia coli W3110

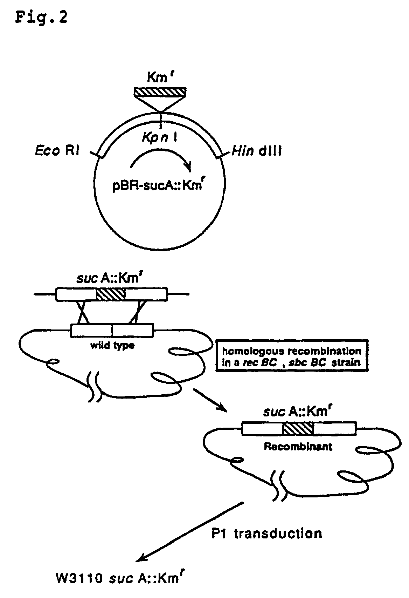

Fig. 2 outlines the disruption of the sucA gene on the chromosomal DNA of Escherichia coli W3110.

pBR-sucAB was digested with KpnI and T4 DNA polymerase was used to obtain blunt ends. On the other hand, pUC4K (purchased from Pharmacia) was digested with PstI to prepare a DNA fragment containing a kanamycin-resistance gene, which was converted to have blunt ends using T4 DNA polymerase. Both fragments were ligated using T4 DNA ligase to obtain pBR-sucA::Km^{r}. From this plasmid, a HindIII-EcoRI fragment containing the kanamycin-resistance gene was cut out as a linear DNA, which was used to transform Escherichia coli JC7623 (thr-1, ara-14, leuB6, Δ(gpt-proA)62, lacY1, tsx-23, supE44, galK2, λ⁻, rac⁻, sbcB15, hisG4, rfbD1, recB21, recC22, rpsL31, kdgK51, xyl-5, mtl-1, argE3, thi-1) obtained from the Escherichia coli Genetic Stock Center (at Yale University, USA), and strains in which the sucA gene on the chromosomal DNA was replaced with the sucA gene into which the kanamycin-resistance gene had been inserted (sucA::Km^{r}) were screened on L medium (bactotrypton 10 g/l, yeast extract 5 g/l, NaCl 5 g/l, agar 15 g/l, pH 7.2) supplemented with 25 µg/ml of kanamycin. Since Escherichia coli JC7623 possessed recB⁻, recC⁻ and sbcB⁻, recombination can be achieved at a high frequency even if the transformation is conducted using a linear DNA.

From each of twelve (12) kanamycin-resistant strains thus obtained, the chromosomal DNA was prepared and digested with KpnI. By southern hybridization using a DNA fragment containing the sucA gene as a probe, all of 12 strains were confirmed to be strains in which the sucA gene on the chromosomal DNA was replaced with the sucA gene into which kanamycin-resistance gene had been inserted. While a wild strain exhibits two bands at 5.2 Kb and 6.2 Kb due to the presence of KpnI site in the DNA fragment containing the sucA gene when a 2.6 Kb EcoRI-HindIII fragment containing the sucA gene of pBR-sucA was used as the probe in the southern hybridization, strains having the replacement with sucA gene into which kanamycin-resistance gene has been inserted exhibits only one band at 11.4 Kb due to the disruption of the KpnI site upon introduction of the kanamycin-resistance gene. The kanamycin-resistance Escherichia coli JC7623 (sucA::Km^{r}) thus obtained was then infected with P1 phage and the phage lysate was prepared. Then Escherichia coli W3100 strain was transduced with the sucA::Km^{r}. Transduction with P1 phage was conducted by a standard method (Seibutsu-kogaku Jikkensho, Ed. by Nippon Seibutsu Kogaku Kai, pp. 75 to 76, Baifukan (1992)). The representative of the kanamycin-resistance strains isolated was designated as W3110 sucA::Km^{r}.

The α-KGDH activities of the strain W3110 sucA::Km^{r} and Escherichia coli W3110 were determined according to the method by Reed et al (Methods in Enzymology, Vol. 13, pp. 55 (1969)). The results are shown in Table 1. α-KGDH activity of Escherichia coli W3110 sucA::Km^{r} was not detected. Thus, Escherichia coli W3110 sucA::Km^{r} is a strain deficient in α-KGDH activity.

**Table 1**

| | W3110 | W3110sucA::Km^{r} |
|---|---|---|
| α-KGDH activity | 3.70 | Not detected |
| (Unit : micromoles/mg protein/min) | | |

### (3) Cloning of gdhA gene of Escherichia coli W3110

Similarly as in the cloning of the sucA and sucB genes, the PCR method was used to clone the gdhA gene. According to the nucleotide sequence of gdhA gene reported by Fernando et al, primers for PCR were synthesized. The nucleotide sequence of the gdhA gene is disclosed in Gene, Vol. 27, pp.193 to 199 (1984), and is also shown here as Sequ ID No. 8 in the sequence listings. The nucleotide sequences of the primers are shown in Sequ ID Nos. 5 and 6.

Sequ ID No. 5 corresponds to the sequence from the 191st through the 171st base residues in the nucleotide sequence figure of gdhA gene shown in Gene, Vol. 27, p.195 (1984), and it also corresponds to the sequence from the 3rd through the 23rd base residues in Sequ ID No. 8.

Sequ ID No. 6 corresponds to the sequence consisting of the 1687th through the 1707th base residues in the nucleotide sequence figure of the gdhA gene shown in Gene, Vol. 27, p.195, (1984), and it also corresponds to the sequence consisting of 1880th through the 1900th base residues in Sequ ID No. 8.

Using the synthetic primers the gdhA gene was amplified by the PCR method employing the chromosomal DNA of Escherichia coli W3110 as a template. PCR products thus obtained were purified and converted to have blunt ends using T4 DNA polymerase, and then ligated to pBR 322 digested with EcoRV to obtain a plasmid pBRGDH.

### (4) Construction of a plasmid having the ppc and gdhA genes

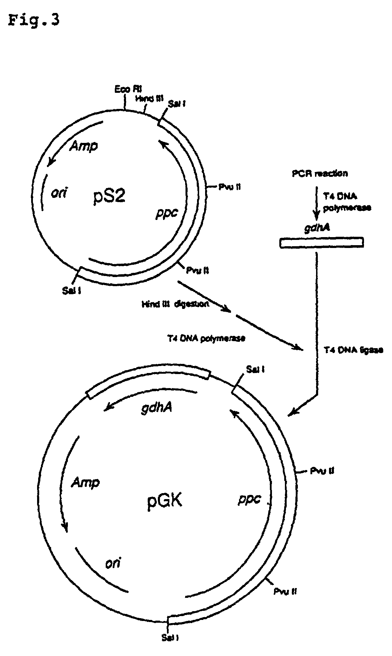

Fig. 3 shows the procedure for the construction of a plasmid having the ppc and gdhA genes. The plasmid pS2 in which 4.4Kb SalI fragment containing the whole region of the ppc gene derived from Escherichia coli K-12 was inserted into the SalI site of pBR322 (J. Biochem, Vol. 9, pp.909 to 916 (1984)) was digested with HindIII and both ends were made blunt using T4 DNA polymerase. On the other hand, a DNA fragment containing the gdhA gene synthesized by the PCR method was converted to have blunt ends using T4 DNA polymerase. Subsequently, both fragments were ligated using T4 DNA ligase. The plasmid thus obtained was used to transform a GDH deficient strain, Escherichia coli PA 340 (thr-1, fhuA2, leuB6, lacY1, supE44, gal-6, λ⁻, gdh-1, hisG1, rfbD1, galP63, Δ(gltB-F), rpsL19, malT1(lambdaR), xyl-7, mtl-2, argH1, thi-1) obtained from the Escherichia coli Genetic Stock Center (at Yale University, USA) and an ampicillin-resistant strain which had lost its glutamic acid requirement for growth was isolated. From this strain, a plasmid was prepared and the restriction map was constructed, whereby it was confirmed that the ppc and gdhA genes were present on this plasmid. This plasmid was designated as pGK.

### (5) Introduction of pS2, pBRGDH and pGK into α-KGDH deficient strain Escherichia coli W3100 sucA::Km^{r} and evaluation of L-glutamic acid-production

The α-KGDH-deficient strain, Escherichia coli W3100 sucA:: Km^{r,} was transformed with each of pS2, pBRGDH and pGK, and each of the transformed strains was inoculated into a 500-ml shaker flask containing 20 ml of the culture medium having the composition shown in Table 2. Cultivation was then carried out at 37 °C until the glucose in the culture medium was consumed completely. The results are shown in Table 3.

**Table 2**

| Component | Concentration (g/l) |
|---|---|
| Glucose | 40 |
| (NH₄)₂SO₄ | 20 |
| KH₂PO₄ | 1 |
| MgSO₄·7H₂O | 1 |
| FeSO₄·7H₂O | 0.01 |
| MnSO₄·5H₂O | 0.01 |
| Yeast extract | 2 |
| Thiamine hydrochloride | 0.01 |
| CaCO₃ | 50 |

**Table 3**

| Strain | Accumulated L-glutamic acid (g/l) |
|---|---|
| W3110 sucA::Km^{r} | 19.2 |
| W3110 sucA::Km^{r}/pS2 | 19.9 |
| W3110 sucA::Km^{r}/pBRGDH | 2.8 |
| W3110 sucA::Km^{r}/pGK (AJ 12949) | 23.3 |

Although the transformed strain having the PPC activity amplified by the introduction of pS2 exhibited slightly reduced growth as compared with the host strain, W3110 sucA::Km^{r}, it accumulated L-glutamic acid in an amount similar to that accumulated by the host strain. The strain having GDH activity amplified by the introduction of pBRGDH exhibited quite poor growth, and the amount of the accumulated L-glutamic acid was surprisingly smaller than that accumulated by the strain W3110 sucA::Km^{r}.

On the contrary, the transformed strain in which both of PPC and GDH activities were amplified simultaneously by the introduction of pGK exhibited growth similar to that of the host strain while producing an increased amount of accumulated L-glutamic acid. Escherichia coli W3110 sucA::Km^{r} into which pGK plasmid having the ppc and gdhA genes had been introduced was designated as AJ 12949. Escherichia coli AJ 12949 was originally deposited under the accession number FERM P-14039 on December 28, 1993, at the National Institute of Bioscience and Human-Technology, Agency of Industrial Science and Technology, 1-3, Higashi 1-chome, Tsukuba-shi, Ibaraki-ken 305, Japan, and the deposit was converted into a deposit under the Budapest Treaty under the accession number FERM BP-4881 on November 11, 1994.

The host strain, namely, W3110 sucA::Km^{r} can be obtained by curing the plasmid from the deposited strain, AJ 12949 without damaging the cell. The plasmid may be lost from AJ 12949 spontaneously, or may be cured in a curing procedure (Bact. Rev., Vol. 36, p.361 to 405 (1972)). An example of the curing procedure is as follows. The strain AJ 12949 is inoculated to the L-broth medium (Bactotrypton 10 g/l, yeast extract 5 g/l, NaCl 5 g/l, pH 7.2), and cultivated at 40°C overnight. The culture broth is diluted appropriately, and spread onto the L-medium. After incubating it at 37° C overnight, the colonies formed are transferred to the L-medium containing 100 µg/ml of ampicillin and then ampicillin-sensitive colonies are isolated. The strain thus obtained is W3110 sucA::Km^{r}.

### Advantages of the Invention

The method according to the present invention provides a mutant of the genus Escherichia having a higher productivity of L-glutamic acid as well as the efficient and low-cost method for the production of L-glutamic acid.

### SEQUENCE LISTING

### GENERAL INFORMATION:

APPLICANT:
NAME: Ajinomoto Co., Inc.
STREET: 15-1, Kyobashi 1-chome
CITY: Chuo-ku, Tokyo
COUNTRY: Japan
POSTAL CODE: none

### TITLE OF INVENTION: Method of producing L-glutamic acid by fermentation

### NUMER OF SEQUENCES: 8

COMPUTER READABLE FORM:
MEDIUM TYPE: Diskette
COMPUTER: IBM PC compatible
OPERATING SYSTEM: MS-DOS

SEQUENCE DESCRIPTION:
SEQ ID No.: 1
Length : 21 base pairs
Type : Nucleotide
Strandedness : Single
Topology : Linear
Molecule type: Synthetic DNA
Feature : Primer for amplification of sucA gene of Escherichia coli

Sequence
ACGCGCAAGC GTCGCATCAG G 21

SEQ ID No.: 2
Length : 21 base pairs
Type : Nucleotide
Strandedness : Single
Topology : Linear
Molecule type: Synthetic DNA
Feature : Primer for amplification of sucA gene of Escherichia coli 
Sequence
ATCGGCTACG AATTCAGGCA G 21
 SEQ ID No.: 3
Length : 20 base pairs
Type : Nucleotide
Strandedness : Single
Topology : Linear
Molecule type: Synthetic DNA
Feature : Primer for amplification of sucB gene of Escherichia coli 
Sequence
CCGGTCGCGG TACCTTCTTC 20
 SEQ ID No.: 4
Length : 26 base pairs
Type : Nucleotide
Strandedness : Single
Topology : Linear
Molecule type: Synthetic DNA
Feature : Primer for amplification of sucB gene of Escherichia coli 
Sequence
CGTAGACCGA ATTCTTCGTA TCGCTT 26
 SQ ID No.: 5
Length : 21 base pairs
Type : Nucleotide
Strandedness : Single
Topology : Linear
Molecule type: Synthetic DNA
Feature : Primer for amplification of gdhA gene of Escherichia coli 
Sequence
GGGTGGCAAA GCTTTAGCGT C 21
 SEQ ID No.: 6
Length : 21 base pairs
Type : Nucleotide
Strandedness : Single
Topology : Linear
Molecule type: Synthetic DNA
Feature : Primer for amplification of gdhA gene of Escherichia coli 
Sequence
TCGAGAAGCA TGCATTATAT A 21
 SEQ ID No.: 7
Length : 4623 base pairs
Type : Nucleotide
Strandedness : Single
Topology : Linear
Molecule type: Genomic DNA
Original source
Organism : Escherichia coli
Features
Feature key : CDS ⇒from 327 to 318 bp coding sequence
Location : 327..3128
Method of feature determination : E
Feature key : CDS ⇒from 3143 to 4357 bp coding sequence Location : 3143..4357
Method of feature determination : E 
Sequence
TCGATGTTGT TGCAACGTAA TGCGTAAACC GTAGGCCTGA TAAGACGCGC AAGCGTCGCA 60
TCAGGCAACC AGTGCCGGAT GCGCGTGAAC GCCTTATCCG GCCTACAAGT CATTACCCGT 120
AGGCCTGATA AGCGCAGCGC ATCAGGCGTA ACAAAGAAAT GCAGGAAATC TTTAAAAACT 180
GCCCCTGACA CTAAGACAGT TTTTAAAGGT TCCTTCGCGA GCCACTACGT AGACAAGAGC 240
TCGCAAGTGA ACCCCGGCAC GCACATCACT GTGCGTGGTA GTATCCACGG CGAAGTAAGC 300
ATAAAAAAGA TGCTTAAGGG ATCACG ATG CAG AAC AGC GCT TTG AAA GCC TGG 353
Met Gln Asn Ser Ala Leu Lys Ala Trp
1               5
TTG GAC TCT TCT TAC CTC TCT GGC GCA AAC CAG AGC TGG ATA GAA CAG 401
Leu Asp Ser Ser Tyr Leu Ser Gly Ala Asn Gln Ser Trp Ile Glu Gln
10                  15                  20                  25
CTC TAT GAA GAC TTC TTA ACC GAT CCT GAC TCG GTT GAC GCT AAC TGG 449
Leu Tyr Glu Asp Phe Leu Thr Asp Pro Asp Ser Val Asp Ala Asn Trp
                30                  35                  40
CGT TCG ACG TTC CAG CAG TTA CCT GGT ACG GGA GTC AAA CCG GAT CAA 497
Arg Ser Thr Phe Gln Gln Leu Pro Gly Thr Gly Val Lys Pro Asp Gln
            45                  50                  55
TTC CAC TCT CAA ACG CGT GAA TAT TTC CGC CGC CTG GCG AAA GAC GCT 545
Phe His Ser Gln Thr Arg Glu Tyr Phe Arg Arg Leu Ala Lys Asp Ala
        60                  65                  70
TCA CGT TAC TCT TCA ACG ATC TCC GAC CCT GAC ACC AAT GTG AAG CAG 593
Ser Arg Tyr Ser Ser Thr Ile Ser Asp Pro Asp Thr Asn Val Lys Gln
    75                  80                  85
GTT AAA GTC CTG CAG CTC ATT AAC GCA TAC CGC TTC CGT GGT CAC CAG 641
Val Lys Val Leu Gln Leu Ile Asn Ala Tyr Arg Phe Arg Gly His Gln
90                  95                  100                 105
CAT GCG AAT CTC GAT CCG CTG GGA CTG TGG CAG CAA GAT AAA GTG GCC 689
His Ala Asn Leu Asp Pro Leu Gly Leu Trp Gln Gln Asp Lys Val Ala
                110                 115                 120
GAT CTG GAT CCG TCT TTC CAC GAT CTG ACC GAA GCA GAC TTC CAG GAG 737
Asp Leu Asp Pro Ser Phe His Asp Leu Thr Glu Ala Asp Phe Gln Glu
            125                 130                 135
ACC TTC AAC GTC GGT TCA TTT GCC AGC GGC AAA GAA ACC ATG AAA CTC 785
Thr Phe Asn Val Gly Ser Phe Ala Ser Gly Lys Glu Thr Met Lys Leu
        140                 145                 150
GGC GAG CTG CTG GAA GCC CTC AAG CAA ACC TAC TGC GGC CCG ATT GGT 833
Gly Glu Leu Leu Glu Ala Leu Lys Gln Thr Tyr Cys Gly Pro Ile Gly
    155                 160                 165
GCC GAG TAT ATG CAC ATT ACC AGC ACC GAA GAA AAA CGC TGG ATC CAA 881
Ala Glu Tyr Met His Ile Thr Ser Thr Glu Glu Lys Arg Trp Ile Gln
170                 175                 180                 185
CAG CGT ATC GAG TCT GGT CGC GCG ACT TTC AAT AGC GAA GAG AAA AAA 929
Gln Arg Ile Glu Ser Gly Arg Ala Thr Phe Asn Ser Glu Glu Lys Lys
                190                 195                 200
CGC TTC TTA AGC GAA CTG ACC GCC GCT GAA GGT CTT GAA CGT TAC CTC 977
Arg Phe Leu Ser Glu Leu Thr Ala Ala Glu Gly Leu Glu Arg Tyr Leu
            205                 210                 215
GGC GCA AAA TTC CCT GGC GCA AAA CGC TTC TCG CTG GAA GGC GGT GAC 1025
Gly Ala Lys Phe Pro Gly Ala Lys Arg Phe Ser Leu Glu Gly Gly Asp
        220                 225                 230
GCG TTA ATC CCG ATG CTT AAA GAG ATG ATC CGC CAC GCT GGC AAC AGC 1073
Ala Leu Ile Pro Met Leu Lys Glu Met Ile Arg His Ala Gly Asn Ser
    235                 240                 245
GGC ACC CGC GAA GTG GTT CTC GGG ATG GCG CAC CGT GGT CGT CTG AAC 1121
Gly Thr Arg Glu Val Val Leu Gly Met Ala His Arg Gly Arg Leu Asn
250                 255                 260                 265
GTG CTG GTG AAC GTG CTG GGT AAA AAA CCG CAA GAC TTG TTC GAC GAG 1169
Val Leu Val Asn Val Leu Gly Lys Lys Pro Gln Asp Leu Phe Asp Glu
                270                 275                 280
TTC GCC GGT AAA CAT AAA GAA CAC CTC GGC ACG GGT GAC GTG AAA TAC 1217
Phe Ala Gly Lys His Lys Glu His Leu Gly Thr Gly Asp Val Lys Tyr
 
            285                 290                 295
CAC ATG GGC TTC TCG TCT GAC TTC CAG ACC GAT GGC GGC CTG GTG CAC 1265
His Met Gly Phe Ser Ser Asp Phe Gln Thr Asp Gly Gly Leu Val His
        300                 305                 310
CTG GCG CTG GCG TTT AAC CCG TCT CAC CTT GAG ATT GTA AGC CCG GTA 1313
Leu Ala Leu Ala Phe Asn Pro Ser His Leu Glu Ile Val Ser Pro Val
    315                 320                 325
GTT ATC GGT TCT GTT CGT GCC CGT CTG GAC AGA CTT GAT GAG CCG AGC 1361
Val Ile Gly Ser Val Arg Ala Arg Leu Asp Arg Leu Asp Glu Pro Ser
330                 335                 340                 345
AGC AAC AAA GTG CTG CCA ATC ACC ATC CAC GGT GAC GCC GCA GTG ACC 1409
Ser Asn Lys Val Leu Pro Ile Thr Ile His Gly Asp Ala Ala Val Thr
                350                 355                 360
GGG CAG GGC GTG GTT CAG GAA ACC CTG AAC ATG TCG AAA GCG CGT GGT 1457
Gly Gln Gly Val Val Gln Glu Thr Leu Asn Met Ser Lys Ala Arg Gly
            365                 370                 375
TAT GAA GTT GGC GGT ACG GTA CGT ATC GTT ATC AAC AAC CAG GTT GGT 1505
Tyr Glu Val Gly Gly Thr Val Arg Ile Val Ile Asn Asn Gln Val Gly
        380                 385                 390
TTC ACC ACC TCT AAT CCG CTG GAT GCC CGT TCT ACG CCG TAC TGT ACT 1553
Phe Thr Thr Ser Asn Pro Leu Asp Ala Arg Ser Thr Pro Tyr Cys Thr
    395                 400                 405
GAT ATC GGT AAG ATG GTT CAG GCC CCG ATT TTC CAC GTT AAC GCG GAC 1601
Asp Ile Gly Lys Met Val Gln Ala Pro Ile Phe His Val Asn Ala Asp
410                 415                 420                 425
GAT CCG GAA GCC GTT GCC TTT GTG ACC CGT CTG GCG CTC GAT TTC CGT 1649
Asp Pro Glu Ala Val Ala Phe Val Thr Arg Leu Ala Leu Asp Phe Arg
                430                 435                 440
AAC ACC TTT AAA CGT GAT GTC TTC ATC GAC CTG GTG TCG TAC CGC CGT 1697
Asn Thr Phe Lys Arg Asp Val Phe Ile Asp Leu Val Ser Tyr Arg Arg
            445                 450                 455
CAC GGC CAC AAC GAA GCC GAC GAG CCG AGC GCA ACC CAG CCG CTG ATG 1745
His Gly His Asn Glu Ala Asp Glu Pro Ser Ala Thr Gln Pro Leu Met
        460                 465                 470
TAT CAG AAA ATC AAA AAA CAT CCG ACA CCG CGC AAA ATC TAC GCT GAC 1793
Tyr Gln Lys Ile Lys Lys His Pro Thr Pro Arg Lys Ile Tyr Ala Asp
    475                 480                 485
AAG CTG GAG CAG GAA AAA GTG GCG ACG CTG GAA GAT GCC ACC GAG ATG 1841
Lys Leu Glu Gln Glu Lys Val Ala Thr Leu Glu Asp Ala Thr Glu Met
490                 495                 500                 505
GTT AAC CTG TAC CGC GAT GCG CTG GAT GCT GGC GAT TGC GTA GTG GCA 1889
Val Asn Leu Tyr Arg Asp Ala Leu Asp Ala Gly Asp Cys Val Val Ala
                510                 515                 520
GAG TGG CGT CCG ATG AAC ATG CAC TCT TTC ACC TGG TCG CCG TAC CTC 1937
Glu Trp Arg Pro Met Asn Met His Ser Phe Thr Trp Ser Pro Tyr Leu
            525                 530                 535
AAC CAC GAA TGG GAC GAA GAG TAC CCG AAC AAA GTT GAG ATG AAG CGC 1985
Asn His Glu Trp Asp Glu Glu Tyr Pro Asn Lys Val Glu Met Lys Arg
        540                 545                 550
CTG CAG GAG CTG GCG AAA CGC ATC AGC ACG GTG CCG GAA GCA GTT GAA 2033
Leu Gln Glu Leu Ala Lys Arg Ile Ser Thr Val Pro Glu Ala Val Glu
    555                 560                 565
ATG CAG TCT CGC GTT GCC AAG ATT TAT GGC GAT CGC CAG GCG ATG GCT 2081
Met Gln Ser Arg Val Ala Lys Ile Tyr Gly Asp Arg Gln Ala Met Ala
570                 575                 580                 585
GCC GGT GAG AAA CTG TTC GAC TGG GGC GGT GCG GAA AAC CTC GCT TAC 2129
Ala Gly Glu Lys Leu Phe Asp Trp Gly Gly Ala Glu Asn Leu Ala Tyr
                590                 595                 600
GCC ACG CTG GTT GAT GAA GGC ATT CCG GTT CGC CTG TCG GGT GAA GAC 2177
Ala Thr Leu Val Asp Glu Gly Ile Pro Val Arg Leu Ser Gly Glu Asp
            605                 610                 615
TCC GGT CGC GGT ACC TTC TTC CAC CGC CAC GCG GTG ATC CAC AAC CAG 2225
 
Ser Gly Arg Gly Thr Phe Phe His Arg His Ala Val Ile His Asn Gln
        620                 625                 630
TCT AAC GGT TCC ACT TAC ACG CCG CTG CAA CAT ATC CAT AAC GGG CAG     2273
Ser Asn Gly Ser Thr Tyr Thr Pro Leu Gln His Ile His Asn Gly Gln
    635                 640                 645
GGC GCG TTC CGT GTC TGG GAC TCC GTA CTG TCT GAA GAA GCA GTG CTG     2321
Gly Ala Phe Arg Val Trp Asp Ser Val Leu Ser Glu Glu Ala Val Leu
650                 655                 660                 665
GCG TTT GAA TAT GGT TAT GCC ACC GCA GAA CCA CGC ACT CTG ACC ATC     2369
Ala Phe Glu Tyr Gly Tyr Ala Thr Ala Glu Pro Arg Thr Leu Thr Ile
                670                 675                 680
TGG GAA GCG CAG TTC GGT GAC TTC GCC AAC GGT GCG CAG GTG GTT ATC     2417
Trp Glu Ala Gln Phe Gly Asp Phe Ala Asn Gly Ala Gln Val Val Ile
            685                 690                 695
GAC CAG TTC ATC TCC TCT GGC GAA CAG AAA TGG GGC CGG ATG TGT GGT     2465
Asp Gln Phe Ile Ser Ser Gly Glu Gln Lys Trp Gly Arg Met Cys Gly
        700                 705                 710
CTG GTG ATG TTG CTG CCG CAC GGT TAC GAA GGG CAG GGG CCG GAG CAC     2513
Leu Val Met Leu Leu Pro His Gly Tyr Glu Gly Gln Gly Pro Glu His
    715                 720                 725
TCC TCC GCG CGT CTG GAA CGT TAT CTG CAA CTT TGT GCT GAG CAA AAC     2561
Ser Ser Ala Arg Leu Glu Arg Tyr Leu Gln Leu Cys Ala Glu Gln Asn
730                 735                 740                 745
ATG CAG GTT TGC GTA CCG TCT ACC CCG GCA CAG GTT TAC CAC ATG CTG     2609
Met Gln Val Cys Val Pro Ser Thr Pro Ala Gln Val Tyr His Met Leu
                750                 755                 760
CGT CGT CAG GCG CTG CGC GGG ATG CGT CGT CCG CTG GTC GTG ATG TCG     2657
Arg Arg Gln Ala Leu Arg Gly Met Arg Arg Pro Leu Val Val Met Ser
            765                 770                 775
CCG AAA TCC CTG CTG CGT CAT CCG CTG GCG GTT TCC AGC CTC GAA GAA     2705
Pro Lys Ser Leu Leu Arg His Pro Leu Ala Val Ser Ser Leu Glu Glu
        780                 785                 790
CTG GCG AAC GGC ACC TTC CTG CCA GCC ATC GGT GAA ATC GAC GAG CTT     2753
Leu Ala Asn Gly Thr Phe Leu Pro Ala Ile Gly Glu Ile Asp Glu Leu
    795                 800                 805
GAT CCG AAG GGC GTG AAG CGC GTA GTG ATG TGT TCT GGT AAG GTT TAT     2801
Asp Pro Lys Gly Val Lys Arg Val Val Met Cys Ser Gly Lys Val Tyr
810                 815                 820                 825
TAC GAC CTG CTG GAA CAG CGT CGT AAG AAC AAT CAA CAC GAT GTC GCC     2849
Tyr Asp Leu Leu Glu Gln Arg Arg Lys Asn Asn Gln His Asp Val Ala
                830                 835                 840
ATT GTG CGT ATC GAG CAA CTC TAC CCG TTC CCG CAT AAA GCG ATG CAG     2897
Ile Val Arg Ile Glu Gln Leu Tyr Pro Phe Pro His Lys Ala Met Gln
            845                 850                 855
GAA GTG TTG CAG CAG TTT GCT CAC GTC AAG GAT TTT GTC TGG TGC CAG     2945
Glu Val Leu Gln Gln Phe Ala His Val Lys Asp Phe Val Trp Cys Gln
        860                 865                 870
GAA GAG CCG CTC AAC CAG GGC GCA TGG TAC TGC AGC CAG CAT CAT TTC     2993
Glu Glu Pro Leu Asn Gln Gly Ala Trp Tyr Cys Ser Gln His His Phe
    875                 880                 885
CGT GAA GTG ATT CCG TTT GGG GCT TCT CTG CGT TAT GCA GGC CGC CCG     3041
Arg Glu Val Ile Pro Phe Gly Ala Ser Leu Arg Tyr Ala Gly Arg Pro
890                 895                 900                 905
GCC TCC GCC TCT CCG GCG GTA GGG TAT ATG TCC GTT CAC CAG AAA CAG     3089
Ala Ser Ala Ser Pro Ala Val Gly Tyr Met Ser Val His Gln Lys Gln
                910                 915                 920
CAA CAA GAT CTG GTT AAT GAC GCG CTG AAC GTC GAA TAAATAAAGG          3135
Gln Gln Asp Leu Val Asn Asp Ala Leu Asn Val Glu
            925                 930
ATACACA ATG AGT AGC GTA GAT ATT CTG GTC CCT GAC CTG CCT GAA TCC     3184
        Met Ser Ser Val Asp Ile Leu Val Pro Asp Leu Pro Glu Ser
          1               5                  10
 
GTA GCC GAT GCC ACC GTC GCA ACC TGG CAT AAA AAA CCC GGC GAC GCA 3232
Val Ala Asp Ala Thr Val Ala Thr Trp His Lys Lys Pro Gly Asp Ala
15 20 25 30
GTC GTA CGT GAT GAA GTG CTG GTA GAA ATC GAA ACT GAC AAA GTG GTA 3280
Val Val Arg Asp Glu Val Leu Val Glu Ile Glu Thr Asp Lys Val Val
35 40 45
CTG GAA GTA CCG GCA TCA GCA GAC GGC ATT CTG GAT GCG GTT CTG GAA 3328
Leu Glu Val Pro Ala Ser Ala Asp Gly Ile Leu Asp Ala Val Leu Glu
50 55 60
GAT GAA GGT ACA ACG GTA ACG TCT CGT CAG ATC CTT GGT CGC CTG CGT 3376
Asp Glu Gly Thr Thr Val Thr Ser Arg Gln Ile Leu Gly Arg Leu Arg
65 70 75
GAA GGC AAC AGC GCC GGT AAA GAA ACC AGC GCC AAA TCT GAA GAG AAA 3424
Glu Gly Asn Ser Ala Gly Lys Glu Thr Ser Ala Lys Ser Glu Glu Lys
80 85 90
GCG TCC ACT CCG GCG CAA CGC CAG CAG GCG TCT CTG GAA GAG CAA AAC 3472
Ala Ser Thr Pro Ala Gln Arg Gln Gln Ala Ser Leu Glu Glu Gln Asn
95 100 105 110
AAC GAT GCG TTA AGC CCG GCG ATC CGT CGC CTG CTG GCT GAA CAC AAT 3520
Asn Asp Ala Leu Ser Pro Ala Ile Arg Arg Leu Leu Ala Glu His Asn
115 120 125
CTC GAC GCC AGC GCC ATT AAA GGC ACC GGT GTG GGT GGT CGT CTG ACT 3568
Leu Asp Ala Ser Ala Ile Lys Gly Thr Gly Val Gly Gly Arg Leu Thr
130 135 140
CGT GAA GAT GTG GAA AAA CAT CTG GCG AAA GCC CCG GCG AAA GAG TCT 3616
Arg Glu Asp Val Glu Lys His Leu Ala Lys Ala Pro Ala Lys Glu Ser
145 150 155
GCT CCG GCA GCG GCT GCT CCG GCG GCG CAA CCG GCT CTG GCT GCA CGT 3664
Ala Pro Ala Ala Ala Ala Pro Ala Ala Gln Pro Ala Leu Ala Ala Arg
160 165 170
AGT GAA AAA CGT GTC CCG ATG ACT CGC CTG CGT AAG CGT GTG GCA GAG 3712
Ser Glu Lys Arg Val Pro Met Thr Arg Leu Arg Lys Arg Val Ala Glu
175 180 185 190
CGT CTG CTG GAA GCG AAA AAC TCC ACC GCC ATG CTG ACC ACG TTC AAC 3760
Arg Leu Leu Glu Ala Lys Asn Ser Thr Ala Met Leu Thr Thr Phe Asn
195 200 205
GAA GTC AAC ATG AAG CCG ATT ATG GAT CTG CGT AAG CAG TAC GGT GAA 3808
Glu Val Asn Met Lys Pro Ile Met Asp Leu Arg Lys Gln Tyr Gly Glu
210 215 220
GCG TTT GAA AAA CGC CAC GGC ATC CGT CTG GGC TTT ATG TCC TTC TAC 3856
Ala Phe Glu Lys Arg His Gly Ile Arg Leu Gly Phe Met Ser Phe Tyr
225 230 235
GTG AAA GCG GTG GTT GAA GCC CTG AAA CGT TAC CCG GAA GTG AAC GCT 3904
Val Lys Ala Val Val Glu Ala Leu Lys Arg Tyr Pro Glu Val Asn Ala
240 245 250
TCT ATC GAC GGC GAT GAC GTG GTT TAC CAC AAC TAT TTC GAC GTC AGC 3952
Ser Ile Asp Gly Asp Asp Val Val Tyr His Asn Tyr Phe Asp Val Ser
255 260 265 270
ATG GCG GTT TCT ACG CCG CGC GGC CTG GTG ACG CCG GTT CTG CGT GAT 4000
Met Ala Val Ser Thr Pro Arg Gly Leu Val Thr Pro Val Leu Arg Asp
275 280 285
GTC GAT ACC CTC GGC ATG GCA GAC ATC GAG AAG AAA ATC AAA GAG CTG 4048
Val Asp Thr Leu Gly Met Ala Asp Ile Glu Lys Lys Ile Lys Glu Leu
290 295 300
GCA GTC AAA GGC CGT GAC GGC AAG CTG ACC GTT GAA GAT CTG ACC GGT 4096
Ala Val Lys Gly Arg Asp Gly Lys Leu Thr Val Glu Asp Leu Thr Gly
305 310 315
GGT AAC TTC ACC ATC ACC AAC GGT GGT GTG TTC GGT TCC CTG ATG TCT 4144
Gly Asn Phe Thr Ile Thr Asn Gly Gly Val Phe Gly Ser Leu Met Ser
320 325 330
ACG CCG ATC ATC AAC CCG CCG CAG AGC GCA ATT CTG GGT ATG CAC GCT 4192
Thr Pro Ile Ile Asn Pro Pro Gln Ser Ala Ile Leu Gly Met His Ala
 
335                 340                 345                 350
ATC AAA GAT CGT CCG ATG GCG GTG AAT GGT CAG GTT GAG ATC CTG CCG     4240
Ile Lys Asp Arg Pro Met Ala Val Asn Gly Gln Val Glu Ile Leu Pro
                355                 360                 365
ATG ATG TAC CTG GCG CTG TCC TAC GAT CAC CGT CTG ATC GAT GGT CGC     4288
Met Met Tyr Leu Ala Leu Ser Tyr Asp His Arg Leu Ile Asp Gly Arg
            370                 375                 380
GAA TCC GTG GGC TTC CTG GTA ACG ATC AAA GAG TTG CTG GAA GAT CCG     4336
Glu Ser Val Gly Phe Leu Val Thr Ile Lys Glu Leu Leu Glu Asp Pro
        385                 390                 395
ACG CGT CTG CTG CTG GAC GTG TAGTAGTTTA AGTTTCACCT GCACTGTAGA     4387
Thr Arg Leu Leu Leu Asp Val
    400                 405
CCGGATAAGG CATTATCGCC TTCTCCGGCA ATTGAAGCCT GATGCGACGC TGACGCGTCT     4447
TATCAGGCCT ACGGGACCAC CAATGTAGGT CGGATAAGGC GCAACGCCGC ATCCGACAAG     4507
CGATGCCTGA TGTGACGTTT AACGTGTCTT ATCAGGCCTA CGGGTGACCG ACAATGCCCG     4567
GAAGCGATAC GAAATATTCG GTCTACGGTT TAAAAGATAA CGATTACTGA AGGATG     4623
 SEQ ID No.: 8
Length : 1937 base pairs
Type : Nucleotide
Strandedness : Single
Topology : Linear
Molecule type: Genomic DNA
Original source
Organism : Escherichia coli
Sequence feature
Feature key : CDS ⇒from 194 to 1537 bp coding sequence Location : 194..1537
Method of feature determination : E

Sequence
CCGGGTGGCA AAACTTTAGC GTCTGAGGTT ATCGCAATTT GGTTATGAGA TTACTCTCGT 60
TATTAATTTG CTTTCCTGGG TCATTTTTTT CTTGCTTACC GTCACATTCT TGATGGTATA 120
GTCGAAAACT GCAAAAGCAC ATGACATAAA CAACATAAGC ACAATCGTAT TAATATATAA 180
GGGTTTTATA TCT ATG GAT CAG ACA TAT TCT CTG GAG TCA TTC CTC AAC 229
Met Asp Gln Thr Tyr Ser Leu Glu Ser Phe Leu Asn
1               5                   10
CAT GTC CAA AAG CGC GAC CCG AAT CAA ACC GAG TTC GCG CAA GCC GTT 277
His Val Gln Lys Arg Asp Pro Asn Gln Thr Glu Phe Ala Gln Ala Val
        15                  20                  25
CGT GAA GTA ATG ACC ACA CTC TGG CCT TTT CTT GAA CAA AAT CCA AAA 325
Arg Glu Val Met Thr Thr Leu Trp Pro Phe Leu Glu Gln Asn Pro Lys
    30                  35                  40
TAT CGC CAG ATG TCA TTA CTG GAG CGT CTG GTT GAA CCG GAG CGC GTG 373
Tyr Arg Gln Met Ser Leu Leu Glu Arg Leu Val Glu Pro Glu Arg Val
45                  50                  55                  60
ATC CAG TTT CGC GTG GTA TGG GTT GAT GAT CGC AAC CAG ATA CAG GTC 421
Ile Gln Phe Arg Val Val Trp Val Asp Asp Arg Asn Gln Ile Gln Val
                65                  70                  75
AAC CGT GCA TGG CGT GTG CAG TTC AGC TCT GCC ATC GGC CCG TAC AAA 469
Asn Arg Ala Trp Arg Val Gln Phe Ser Ser Ala Ile Gly Pro Tyr Lys
            80                  85                  90
GGC GGT ATG CGC TTC CAT CCG TCA GTT AAC CTT TCC ATT CTC AAA TTC 517
Gly Gly Met Arg Phe His Pro Ser Val Asn Leu Ser Ile Leu Lys Phe
        95                  100                 105
CTC GGC TTT GAA CAA ACC TTC AAA AAT GCC CTG ACT ACT CTG CCG ATG 565
Leu Gly Phe Glu Gln Thr Phe Lys Asn Ala Leu Thr Thr Leu Pro Met
    110                 115                 120
GGC GGT GGT AAA GGC GGC AGC GAT TTC GAT CCG AAA GGA AAA AGC GAA 613
Gly Gly Gly Lys Gly Gly Ser Asp Phe Asp Pro Lys Gly Lys Ser Glu

125                    130                    135                    140
GGT GAA GTG ATG CGT TTT TGC CAG GCG CTG ATG ACT GAA CTG TAT CGC          661
Gly Glu Val Met Arg Phe Cys Gln Ala Leu Met Thr Glu Leu Tyr Arg
                    145                    150                    155
CAC CTG GGC GCG GAT ACC GAC GTT CCG GCA GGT GAT ATC GGG GTT GGT          709
His Leu Gly Ala Asp Thr Asp Val Pro Ala Gly Asp Ile Gly Val Gly
                160                    165                    170
GGT CGT GAA GTC GGC TTT ATG GCG GGG ATG ATG AAA AAG CTC TCC AAC          757
Gly Arg Glu Val Gly Phe Met Ala Gly Met Met Lys Lys Leu Ser Asn
            175                    180                    185
AAT ACC GCC TGC GTC TTC ACC GGT AAG GGC CTT TCA TTT GGC GGC AGT          805
Asn Thr Ala Cys Val Phe Thr Gly Lys Gly Leu Ser Phe Gly Gly Ser
        190                    195                    200
CTT ATT CGC CCG GAA GCT ACC GGC TAC GGT CTG GTT TAT TTC ACA GAA          853
Leu Ile Arg Pro Glu Ala Thr Gly Tyr Gly Leu Val Tyr Phe Thr Glu
205                    210                    215                    220
GCA ATG CTA AAA CGC CAC GGT ATG GGT TTT GAA GGG ATG CGC GTT TCC          901
Ala Met Leu Lys Arg His Gly Met Gly Phe Glu Gly Met Arg Val Ser
                    225                    230                    235
GTT TCT GGC TCC GGC AAC GTC GCC CAG TAC GCT ATC GAA AAA GCG ATG          949
Val Ser Gly Ser Gly Asn Val Ala Gln Tyr Ala Ile Glu Lys Ala Met
                240                    245                    250
GAA TTT GGT GCT CGT GTG ATC ACT GCG TCA GAC TCC AGC GGC ACT GTA          997
Glu Phe Gly Ala Arg Val Ile Thr Ala Ser Asp Ser Ser Gly Thr Val
            255                    260                    265
GTT GAT GAA AGC GGA TTC ACG AAA GAG AAA CTG GCA CGT CTT ATC GAA          1045
Val Asp Glu Ser Gly Phe Thr Lys Glu Lys Leu Ala Arg Leu Ile Glu
        270                    275                    280
ATC AAA GCC AGC CGC GAT GGT CGA GTG GCA GAT TAC GCC AAA GAA TTT          1093
Ile Lys Ala Ser Arg Asp Gly Arg Val Ala Asp Tyr Ala Lys Glu Phe
285                    290                    295                    300
GGT CTG GTC TAT CTC GAA GGC CAA CAG CCG TGG TCT CTA CCG GTT GAT          1141
Gly Leu Val Tyr Leu Glu Gly Gln Gln Pro Trp Ser Leu Pro Val Asp
                    305                    310                    315
ATC GCC CTG CCT TGC GCC ACC CAG AAT GAA CTG GAT GTT GAC GCC GCG          1189
Ile Ala Leu Pro Cys Ala Thr Gln Asn Glu Leu Asp Val Asp Ala Ala
                320                    325                    330
CAT CAG CTT ATC GCT AAT GGC GTT AAA GCC GTC GCC GAA GGG GCA AAT          1237
His Gln Leu Ile Ala Asn Gly Val Lys Ala Val Ala Glu Gly Ala Asn
            335                    340                    345
ATG CCG ACC ACC ATC GAA GCG ACT GAA CTG TTC CAG CAG GCA GGC GTA          1285
Met Pro Thr Thr Ile Glu Ala Thr Glu Leu Phe Gln Gln Ala Gly Val
        350                    355                    360
CTA TTT GCA CCG GGT AAA GCG GCT AAT GCT GGT GGC GTC GCT ACA TCG          1333
Leu Phe Ala Pro Gly Lys Ala Ala Asn Ala Gly Gly Val Ala Thr Ser
365                    370                    375                    380
GGC CTG GAA ATG CCA CAA AAC GCT GCG CGC CTG GGC TGG AAA GCC GAG          1381
Gly Leu Glu Met Pro Gln Asn Ala Ala Arg Leu Gly Trp Lys Ala Glu
                    385                    390                    395
AAA GTT GAC GCA CGT TTG CAT CAC ATC ATG CTG GAT ATC CAC CAT GCC          1429
Lys Val Asp Ala Arg Leu His His Ile Met Leu Asp Ile His His Ala
                400                    405                    410
TGT GTT GAG CAT GGT GGT GAA GGT GAG CAA ACC AAC TAC GTG CAG GGC          1477
Cys Val Glu His Gly Gly Glu Gly Glu Gln Thr Asn Tyr Val Gln Gly
            415                    420                    425
GCG AAC ATT GCC GGT TTT GTG AAG GTT GCC GAT GCG ATG CTG GCG CAG          1525
Ala Asn Ile Ala Gly Phe Val Lys Val Ala Asp Ala Met Leu Ala Gln
        430                    435                    440
GGT GTG ATT TAAGTTGTAA ATGCCTGATG GCGCTACGCT TATCAGGCCT          1574
Gly Val Ile
445
ACAAATGGGC ACAATTCATT GCAGTTACGC TCTAATGTAG GCCGGGCAAG CGCAGCGCCC          1634

CCGGCAAAAT TTCAGGCGTT TATGAGTATT TAACGGATGA TGCTCCCCAC GGAACATTTC 1694
TTATGGGCCA ACGGCATTTC TTACTGTAGT GCTCCCAAAA CTGCTTGTCG TAACGATAAC 1754
ACGCTTCAAG TTCAGCATCC GTTAACTTTC TGCGGACTCA CGCGCGCAGC ACTATGCCAG 1814
TAAAGAAATC CCATTTGACT ATTTTTTTGA TAATCTTCTT CGCTTTCGAA CAACTCGTGC 1874
GCCTTTCGAG AAGCAAGCAT TATATAATGC CAGGCCAGTT CTTCTTCAAT TGTCCCGTTT 1934
TGA 1937

## Claims

1. A mutant of the genus Escherichia having L-glutamic acid-productivity, said mutant having deficient or reduced α-ketoglutarate dehydrogenase activity and enhanced phosphoenolpyruvate carboxylase and glutamate dehydrogenase activities.

2. A method of producing L-glutamic acid by fermentation comprising culturing in a liquid culture medium a mutant of the genus Escherichia having L-glutamic acid-productivity said mutant having deficient or reduced α-ketoglutarate dehydrogenase activity and enhanced phosphoenolpyruvate carboxylase and glutamate dehydrogenase activities, accumulating L-glutamic acid in the culture, and recovering L-glutamic acid therefrom.

## Patentansprüche

1. Mutante der Gattung Escherichia, die L-Glutaminsäure produziert und keine oder verminderte α-Ketoglutaratdehydrogenaseaktivität und erhöhte Phosphoenolpyruvatcarboxylase- und Glutamatdehydrogenaseaktivitäten hat.

2. Verfahren zur Herstellung von L-Glutaminsäure durch Fermentation, welches das Kultivieren einer Mutante der Gattung Escherichia, die L-Glutaminsäure produziert und keine oder verminderte α-Ketoglutaratdehydrogenaseaktivität und erhöhte Phosphoenolpyruvatcarboxylase- und Glutamatdehydrogenaseaktivitäten hat, in einem flüssigen Kulturmedium, das Anhäufen von L-Glutaminsäure in der Kultur und das Gewinnen von L-Glutaminsäure daraus umfaßt.

## Revendications

1. Mutant du genre Escherichia ayant une productivité d'acide L-glutamique, ledit mutant ayant une activité α-cétoglutarate déshydrogénase déficiente ou réduite et des activités phosphoénolpyruvate carboxylase et glutamate déshydrogénase augmentées.

2. Procédé de production d'acide L-glutamique par fermentation comprenant la culture dans un milieu de culture liquide d'un mutant du genre Escherichia ayant une productivité d'acide L-glutamique, ledit mutant ayant une activité α-cétoglutarate déshydrogénase déficiente ou réduite et des activités phosphoénolpyruvate carboxylase et glutamate déshydrogénase augmentées, l'accumulation d'acide L-glutamique dans la culture et la récupération de l'acide L-glutamique à partir de celle-ci.
